# EUROPEAN PATENT SPECIFICATION

(11) **EP 1 581 238 B1**
(45) Date of publication and mention of the grant of the patent: **21.05.2008**
(21) Application number: 03701291.1
(22) Date of filing: 09.01.2003
(51) Int. Cl.: A61K 36/00, A61P 37/04, A61P 31/12, A61P 35/00

(54) **IMPROVEMENTS IN OR RELATING TO IMMUNE FUNCTIONS**
ZYTOTOXISCHE PHARMAZEUTISCHE ZUSAMMENSETZUNG
AMELIORATIONS APPORTEES AUX FONCTIONS IMMUNITAIRES OU RELATIVES A CELLES-CI

(43) Date of publication of application: 05.10.2005
(73) Proprietor: Farley, Michael D., Satellite Beach, FL 32937 (US)
(72) Inventor: Farley, Michael D., Satellite Beach, FL 32937 (US)
(74) Representative: Hoarton, Lloyd Douglas Charles
(86) International application number: PCT/US2003/000709
(87) International publication number: WO 2004/062683

(56) References cited:
- EP-A- 0 742 012
- US-A- 6 086 915
- US-A1- 2002 192 310
- US-B1- 6 238 673
- DE CLERCQ E: "Current lead natural products for the chemotherapy of human immunodeficiency virus (HIV) infection." MEDICINAL RESEARCH REVIEWS. UNITED STATES SEP 2000, vol. 20, no. 5, September 2000 (2000-09), pages 323-349, XP009008036 ISSN: 0198-6325
- BRINKWORTH R I ET AL: "FLAVONES ARE INHIBITORS OF HIV-1 PROTEINASE" BIOCHEMICAL AND BIOPHYSICAL RESEARCH COMMUNICATIONS, ACADEMIC PRESS INC. ORLANDO, FL, US, vol. 188, no. 2, 30 October 1992 (1992-10-30), pages 631-637, XP001031188 ISSN: 0006-291X
- STEERENBERG P A ET AL: "Protection of UV-induced suppression of skin contact hypersensitivity: A common feature of flavonoids after oral administration?" PHOTOCHEMISTRY AND PHOTOBIOLOGY, vol. 67, no. 4, April 1998 (1998-04), pages 456-461, XP009008037 ISSN: 0031-8655

## Description

This invention relates to an immune system of a human body, against Aids, viral and related infectious disease and cancer.

This immune function is a system that functions to include absorption of excess fluid and its return to the blood stream, absorption of fat and finally to the immune system function.

Immunity is the body's capability to repel foreign substances and cells. The non specific responses are the first line of defense. Highly specific responses are the second line of defenses and are tailored to an individual threat.

Accordingly to the present invention, a nutraceutical or a pharmaceutical composition per unit dose in liquid or capsule gel caplets, characterized in that each unit dose consists of:
(a) 200 to 600 mg. of Chrysin;
(b) 200 to 600 mg. of Coriolus Versicolor (PSK);
(c) 50 to 150 mg. of 3,3' Diindolymethane Dim;
(d) 50 to 150 mg. of Resveratrol 25%;
(e) 50 to 150 mg. of Turmeric Extract 95%;
(f) 40 to 140 mg. of Green Tea Extract 95%;
(g) 20 to 80 mg. of Quercitin Dihydrate 99%; and
(h) 15 to 75 mg. of Phosphtidyl Choline 50%.
Preferably the invention may also include 25 mg. to about 150 mg. of Myricetin.

The present invention, advantageously may include a nutraceutical or a pharmaceutical composition per unit dose in liquid or capsule gel caplets characterized in that each dose per unit consists of:
(a) 200 to 600 mg. Chrysin;
(b) 200 to 600 mg. Coriolus Versicolor (PSK);
(c) 50 to 150 mg. 3,3' Diindolymethane Dim;
(d) 50 to 150 mg. Resveratrol 25%;
(e) 50 to 150 mg. Turmeric Extract 95 %;
(f) 40 to 140 mg. Green Tea Extract 95 %;
(g) 20 to 80 mg. Quercitin Dihydrate 99%;
(h) 15 to 75 mg. Phosphtidyl Choline 50%; and
(i) 25 to 150 mg. of Myricetin.

These all natural formulas contain phytochemicals that have been shown to cause cell apoptosis, cytotoxicity and inhibition of replication in all of the following cancer cell lines.
THP - 1 human monocytic leukaemia cells
CaCo-2 human colon cancer cells
Human leukaemia HL - 60 cells
HLA B40 - positive breast cancer cells
Estrogen receptor positive MCF-7 (human breast cancer cell lines)
Estrogen receptor negative MDA-MB-468 (human breast cancer cell lines)
Squamous cell carcinoma (SCC) (oral)
Androgen-sensitive LNCaP (human prostate)
Androgen-insensitive PC-3 cell lines (human prostate)

These phytochemicals have also been tested and found to be effective against Aids, HIV and Herpes viruses. It functions on two levels. On one level it acts directly and positively on the immune system. It does this be increasing the number and function of the body's own natural killer cells as well as by increasing the number and function of lymphocytes. For this reason, it may be one of the most effective prophylactics against Aids, cancer and viral infections available on the market.

The phytochemicals are suspended in liposome's to enchance bioavailability. This same technology is currently being used to enhance the efficacy of some chemotherapeutic agents. It offers a much more direct presentation of the desired phytochemicals to the lymphatic system. It also is used in the treatment of Aids patients.

However, the present invention plays a totally new role to stabilize and form within a body's natural immune function system an enhanced defense mechanism against Aids, viral infectious disease and cancer.

The composition per unit dose in liquid form is given as a normal dosage of two tablespoons three times daily. The dose per unit besides being in liquid form, may also be converted into capsule gel caplets, making it easier to give to a patient, in lieu of the liquid dosage.

An inventive and proprietary formula to enhance the body's natural immune function against Aids, viral and infectious diseases and cancer. The composition per unit dose in liquid or capsule gel caplets consists of:
(a) 200 to 600 mg. Chrysin;
(b) 200 to 600 mg. Coriolus Versicolor PSK;
(c) 50 to 150 mg. of 3,3' Diindolymethane Dim;
(d) 50 to 150 mg. of Resveratrol 25%;
(e) 50 to 150 mg. of Turmeric Extract 95%;
(f) 40 to 140 mg. of Green Tea Extract 95:
(g) 20 to 80 mg. of Quercitin Dihydrate 99%; and
(h) 15 to 75 mg. of Phosphtidyl Choline 50%.
Advantageously, each dose unit preferably includes 25 mg. to about 150 mg. of Myricetin.

In the present invention, the initial test results for some number of person trail, resulted in 75% averaged 92-66 % drop in viral load within a short time of about 42 to 45 days.

All patients, that were treated experienced considerable increase in CD4 and CD8 cells.

## Claims

1. A pharmaceutical composition or a nutraceutical composition per unit dose in liquid form or capsule gel caplets, wherein each unit dose form comprises:
(a) 200 to 600 mg. of Chrysin;
(b) 200 to 600 mg. of Coriolus Versicolor PSK;
(c) 50 to 150 mg. of 3,3' Diindolymethan Dim;
(d) 50 to 150 mg. of Resveratrol 25%;
(e) 50 to 150 mg. Turmeric Extract 95%;
(f) 40 to 140 mg. of Green Tea Extract 95%;
(g) 20 to 80 mg. of Quercitin Dihydrate 99%; and
(h) 15 to 75 mg. of Phosphitidyl Choline 50%.

2. A composition according to Claim 1, wherein each unit dose also includes 25 mg. to about 150 mg. of Myricetin.

3. A composition according to Claim 1, wherein each unit dose form comprises:
(a) 400 mg. Chrysin;
(b) 400 mg. Coriolus Versicolor (PSK);
(c) 100 mg. 3,3' Diindolymethane Dim;
(d) 100 mg. Resveratrol 25%;
(e) 100 mg. Turmeric Extract 95%;
(f) 90 mg. Green Tea Extract 95%;
(a) 50 mg. Quercitin Dihydrate 99%; and
(b) 40 mg. Phosphtidyl Choline 50%.

4. A composition per unit dose in liquid or capsule gel caplets according to Claim 2, wherein each unit dose form comprises:
(a) 400 mg. Chrysin;
(b) 400 mg. Coriolus Versicolor (PSK);
(c) 100 mg. 3,3' Diindolymethane Dim;
(d) 100 mg. Resveratrol 25%;
(e) 100 mg. Turmeric Extract 95%;
(f) 90 mg. Green Tea Extract;
(g) 50 mg. Quercitin Dihydrate 99%;
(h) 40 mg. Phosphtidyl Choline 50%; and
(i) 100 mg. Myricetin.

5. A pharmaceutical or a nutraceutical composition per unit dose in liquid or capsule gel caplets, wherein each dose per unit comprises:
(a) 200 to 600 mg. Chrysin;
(b) 200 to 600 mg. Coriolus Versicolor PSK;
(c) 50 to 150 mg. 3,3' Diindolymethane (DIM);
(d) 50 TO 150 mg. Resveratrol 25%;
(e) 50 to 150 mg. Turmeric Extract 95%;
(f) 40 to 140 mg. Green Tea Extract 95%;
(g) 20 to 80 mg. Quercitin Dihydrate 99%;
(h) 15 to 75 mg. Phosphtidyl Choline 50% and
(i) 25 mg. to about 150 mg. of Myricetin.

6. A pharmaceutical composition or a nutraceutical composition per unit dose in liquid or capsule gel caplets wherein each dose unit comprises:
(a) 400 mg. Chrysin;
(b) 400 mg. Coriolus Versicolor PSK;
(c) 100 mg. 3,3' Diindolymethane Dim;
(d) 100 mg. Resveratrol 25%;
(e) 100 mg. Turmeric Extract 95%;
(f) 90 mg. Green Tea Extract 95%;
(g) 50 mg. Quercitin Dihydrate 99%;
(h) 40 mg. Phosphtidyl Choline 50%; and
(i) 100 mg. Myricetin.

7. A composition according to any one of claims 1 to 6 for use in the treatment of cancer.

8. A composition according to any one of claims 1 to 6 for use in the treatment of AIDS.

9. A composition according to any one of claims 1 to 6 for use in the treatment of viral infectious diseases.

10. Use of a composition according to any one of claims 1 to 6 for the manufacture of a medicament for the treatment of cancer.

11. Use of a composition according to any one of claims 1 to 6 for the manufacture of a medicament for the treatment of AIDS.

12. Use of a composition according to any one of claims 1 to 6 for the manufacture of a medicament for the treatment of viral infectious diseases.

## Patentansprüche

1. Pharmazeutische Zusammensetzung oder Nutraceutical-Zusammensetzung pro Dosiseinheit in flüssiger Form oder Kapselgelcaplets, wobei jede Dosiseinheitsform umfasst:
(a) 200 bis 600 mg Chrysin;
(b) 200 bis 600 mg Coriolus Versicolor PSK;
(c) 50 bis 150 mg 3,3'-Diindolylmethan Dim;
(d) 50 bis 150 mg Resveratrol 25%;
(e) 50 bis 150 mg Kurkumaextrakt 95%;
(f) 40 bis 140 mg Grünteeextrakt 95%;
(g) 20 bis 80 mg Quercitin-Dihydrat 99%; und
(h) 15 bis 75 mg Phosphitidylcholin 50%.

2. Zusammensetzung nach Anspruch 1, **dadurch gekennzeichnet, dass** jede Dosiseinheit auch 25 mg bis etwa 150 mg Myricetin einschließt.

3. Zusammensetzung nach Anspruch 1, **dadurch gekennzeichnet, dass** jede Dosiseinheitsform umfasst:
(a) 400 mg Chrysin;
(b) 400 mg Coriolus Versicolor (PSK);
(c) 100 mg 3,3'-Diindolylmethan Dim;
(d) 100 mg Resveratrol 25%;
(e) 100 mg Kurkumaextrakt 95%;
(f) 90 mg Grünteeextrakt 95%;
(a) 50 mg Quercitin-Dihydrat 99%; und
(b) 40 mg Phosphitidylcholin 50%.

4. Zusammensetzung pro Dosiseinheit in Flüssigkeit oder Kapselgelcaplets nach Anspruch 2, **dadurch gekennzeichnet, dass** jede Dosiseinheitsform umfasst:
(a) 400 mg Chrysin;
(b) 400 mg Coriolus Versicolor (PSK);
(c) 100 mg 3,3'-Diindolylmethan Dim;
(d) 100 mg Resveratrol 25%;
(e) 100 mg Kurkumaextrakt 95%;
(f) 90 mg Grünteeextrakt;
(g) 50 mg Quercitin-Dihydrat 99%;
(h) 40 mg Phosphitidylcholin 50%; und
(i) 100 mg Myricetin.

5. Pharmazeutische oder Nutraceutical-Zusammensetzung pro Dosiseinheit in Flüssigkeit oder Kapselgelcaplets, **dadurch gekennzeichnet, dass** jede Dosis pro Einheit umfasst:
(a) 200 bis 600 mg Chrysin;
(b) 200 bis 600 mg Coriolus Versicolor PSK;
(c) 50 bis 150 mg 3,3'-Diindolylmethan (DIM);
(d) 50 bis 150 mg Resveratrol 25%,
(e) 50 bis 150 mg Kurkumaextrakt 95%;
(f) 40 bis 140 mg Grünteeextrakt 95%;
(g) 20 bis 80 mg Quercitin-Dihydrat 99%;
(h) 15 bis 75 mg Phosphitidylcholin 50%; und
(i) 25 mg bis etwa 150 mg Myricetin.

6. Pharmazeutische Zusammensetzung oder Nutraceutical-Zusammensetzung pro Dosiseinheit in Flüssigkeit oder Kapselgelcaplets, **dadurch gekennzeichnet, dass** jede Dosiseinheit umfasst:
(a) 400 mg Chrysin;
(b) 400 mg Coriolus Versicolor PSK;
(c) 100 mg 3,3'-Diindolylmethan Dim;
(d) 100 mg Resveratrol 25%;
(e) 100 mg Kurkumaextrakt 95%;
(f) 90 mg Grünteeextrakt 95%;
(g) 50 mg Quercitin-Dihydrat 99%;
(h) 40 mg Phosphitidylcholin 50%; und
(i) 100 mg Myricetin.

7. Zusammensetzung nach einem der Ansprüche 1 bis 6 zur Verwendung bei der Behandlung von Krebs.

8. Zusammensetzung nach einem der Ansprüche 1 bis 6 zur Verwendung bei der Behandlung von AIDS.

9. Zusammensetzung nach einem der Ansprüche 1 bis 6 zur Verwendung bei der Behandlung von Virusinfektionserkrankungen.

10. Verwendung einer Zusammensetzung nach einem der Ansprüche 1 bis 6 zur Herstellung eines Arzneimittels zur Behandlung von Krebs.

11. Verwendung einer Zusammensetzung nach einem der Ansprüche 1 bis 6 zur Herstellung eines Arzneimittels zur Behandlung von AIDS.

12. Verwendung einer Zusammensetzung nach einem der Ansprüche 1 bis 6 zur Herstellung eines Arzneimittels zur Behandlung von Virusinfektionskrankheiten.

## Revendications

1. Composition pharmaceutique ou composition nutraceutique par dose unitaire sous forme de liquide ou de comprimés de gel en capsule, dans laquelle chaque forme de dose unitaire comprend :
(a) 200 à 600 mg de chrysine ;
(b) 200 à 600 mg de coriolus versicolor PSK ;
(c) 50 à 150 mg de 3,3'-diindolyméthane DIM ;
(d) 50 à 150 mg de resvératrol à 25 % ;
(e) 50 à 150 mg d'extrait de curcuma à 95 % ;
(f) 40 à 140 mg d'extrait de thé vert à 95 % ;
(g) 20 à 80 mg de dihydrate de quercétine à 99 % ; et
(h) 15 à 75 mg de phosphitidylcholine à 50 %.

2. Composition selon la revendication 1, dans laquelle chaque dose unitaire comprend également de 25 mg à 150 mg de myricétine.

3. Composition selon la revendication 1, dans laquelle chaque forme de dose unitaire comprend :
(a) 400 mg de chrysine ;
(b) 400 mg de coriolus versicolor (PSK) ;
(c) 100 mg de 3,3'-diindolyméthane DIM ;
(d) 100 mg de resvératrol à 25 % ;
(e) 100 mg d'extrait de curcuma à 95 % ;
(f) 90 mg d'extrait de thé vert à 95 % ;
(g) 50 mg de dihydrate de quercétine à 99 % ; et
(h) 40 mg de phosphitidylcholine à 50 %.

4. Composition par dose unitaire sous forme de liquide ou de comprimés de gel en capsule selon la revendication 2, dans laquelle chaque forme de dose unitaire comprend :
(a) 400 mg de chrysine ;
(b) 400 mg de coriolus versicolor (PSK) ;
(c) 100 mg de 3,3'-diindolyméthane DIM ;
(d) 100 mg de resvératrol à 25 % ;
(e) 100 mg d'extrait de curcuma à 95 % ;
(f) 90 mg d'extrait de thé vert à 95 % ;
(g) 50 mg de dihydrate de quercétine à 99 % ;
(h) 40 mg de phosphitidylcholine à 50 % ; et
(i) 100 mg de myricétine.

5. Composition pharmaceutique ou nutraceutique par dose unitaire sous forme de liquide ou de comprimés de gel en capsule, dans laquelle chaque forme de dose unitaire comprend :
(a) 200 à 600 mg de chrysine ;
(b) 200 à 600 mg de coriolus versicolor PSK ;
(c) 50 à 150 mg de 3,3'-diindolyméthane DIM ;
(d) 50 à 150 mg de resvératrol à 25 % ;
(e) 50 à 150 mg d'extrait de curcuma à 95 % ;
(f) 40 à 140 mg d'extrait de thé vert à 95 % ;
(g) 20 à 80 mg de dihydrate de quercétine à 99 % ;
(h) 15 à 75 mg de phosphitidylcholine à 50 % ; et
(i) 25 mg à environ 150 mg de myricétine.

6. Composition pharmaceutique ou composition nutraceutique par dose unitaire sous forme de liquide ou de comprimés de gel en capsule, dans laquelle chaque forme de dose unitaire comprend :
(a) 400 mg de chrysine ;
(b) 400 mg de coriolus versicolor PSK ;
(c) 100 mg de 3,3'-dündolyméthane DIM ;
(d) 100 mg de resvératrol à 25 % ;
(e) 100 mg d'extrait de curcuma à 95 % ;
(f) 90 mg d'extrait de thé vert à 95 % ;
(g) 50 mg de dihydrate de quercétine à 99 % ;
(h) 40 mg de phosphitidylcholine à 50 % ; et
(i) 100 g de myricétine.

7. Composition selon l'une quelconque des revendications 1 à 6, destinée à être utilisée dans le traitement du cancer.

8. Composition selon l'une quelconque des revendications 1 à 6, destinée à être utilisée dans le traitement du SIDA.

9. Composition selon l'une quelconque des revendications 1 à 6, destinée à être utilisée dans le traitement de maladies infectieuses virales.

10. Utilisation d'une composition selon l'une quelconque des revendications 1 à 6 dans la fabrication d'un médicament destiné au traitement du cancer.

11. Utilisation d'une composition selon l'une quelconque des revendications 1 à 6 dans la fabrication d'un médicament destiné au traitement du SIDA.

12. Utilisation d'une composition selon l'une quelconque des revendications 1 à 6 dans la fabrication d'un médicament destiné au traitement de maladies infectieuses virales.
